# EUROPÄISCHE PATENTANMELDUNG

(11) **EP 3 574 857 A1**
(43) Veröffentlichungstag der Anmeldung: **04.12.2019**
(21) Anmeldenummer: 19170970.8
(22) Anmeldetag: 25.04.2019
(51) Int. Cl.: A61B 17/43

(54) **VORRICHTUNG ZUR INTRACERVICALEN INSEMINATION**

(30) Priorität: 28.05.2018 DE 202018102960 U; 13.06.2018 DE 202018103340 U
(71) Anmelder: Consilium Beratungs-Beteiligungs-GmbH, 81479 München (DE)
(72) Erfinder: Bleichrodt, Wolf-Heinrich, 81470 München (DE)
(74) Vertreter: Kruspig, Volkmar

(57) **Zusammenfassung**

Die Erfindung betrifft eine Vorrichtung zur intracervicalen Insemination, bestehend aus einer schalenförmigen, auf den Gebärmutterhals aufsetzbaren Kappe sowie mit einer mit der Kappe verbundenen, von dieser aufsteigenden Lanze zur Aufnahme und Führung von Sperma in den Gebärmutterhals, wobei die Kappe aus einem elastischen Kunststoffmaterial besteht und am zur Kappe weisenden Ende der Lanze in der Kappe eine integrale Ausbauchung zur Bildung eines Spermareservoirs vorgesehen ist, die Lanze mit ihrem kappenseitigen Ende den Ausbauchungs-Reservoirraum unter Freilassung eines Spaltes umgreift und der Spalt in Längsrichtung bis zum kappenfernen Ende zur Führung und zum Austritt von Sperma ausgebildet ist derart, dass Spermien unter Nutzung des vom Zervikalmukus ausgehenden kapillaren Sogs in den Gebärmutterhals diffundieren.

## Beschreibung

Die Erfindung betrifft eine Vorrichtung zur intracervicalen Insemination, bestehend aus einer schalenförmigen, auf den Gebärmutterhals aufsetzbaren Kappe sowie mit einer mit der Kappe verbundenen, von dieser aufsteigenden Lanze zur Aufnahme und Führung von Sperma in den Gebärmutterhals gemäß Anspruch 1.

Aus der US 2 764 975 ist eine Zervikalkappe vorbekannt, welche einen schalenförmigen Körper aus einem flexiblen Material mit einer offenen Oberseite umfasst, die so angepasst ist, dass sie auf den Gebärmutterhals einer Patientin aufsetzbar ist. Der Zervikalkappe umfasst eine kreisförmige Öffnung, die an einem zentralen Abschnitt ausgebildet ist. Weiterhin ist eine längliche, lanzenartige, hohle Spermakammer mit kreisförmigem Querschnitt vorhanden, die lösbar an der Öffnung fixiert werden kann. Diese Spermakammer ist mit dem Boden der Kappe verbunden und erstreckt sich durch die Cervixöffnung in den Uterus. Weiterhin ist die Spermakammer mit einer Rohrführung und einer Pumpanordnung in Verbindung stehend, um Flüssigkeiten injizieren zu können.

Die künstliche Besamungsvorrichtung nach US 2011/0152606 A1 umfasst ein Kondom mit einer Hülle und einer verstärkten Schale, wobei die Schale ein Ende der Hülle abdeckt und eine innere konkave Fläche sowie eine äußere konvexe Oberfläche besitzt. Weiterhin verfügt die Vorrichtung über einen Abgabehandgriff, der eine Verlängerung umfasst, die so bemessen und geformt ist, dass sie die äußere konvexe Oberfläche der Kondomschale berührt. Weiterhin zeigt die US 2011/0152606 A1 ein deformierbares Reservoir, wobei der Abgabegriff einen Reservoirinverter umfasst, der so geformt ist, dass er das deformierbare Reservoir formseitig umkehrt, wenn der Inverter relativ zum Kondom vorgeschoben wird. Hierdurch wird der Inhalt des Kondombechers ausgestoßen.

Bei der künstlichen Besamungsvorrichtung nach US 5 536 243 A wird wiederum von einer Zervikalkappe ausgegangen, die sich dem Gebärmutterhals anpasst und daran haftet. Die Vorrichtung umfasst einen länglichen Nippel, der sich senkrecht von der Kappe aus erstreckt, um in den Zervikalkanal bzw. die Gebärmutter eingeführt werden zu können. Weiterhin ist ein Mechanismus zur zeitlichen Freigabe vorgesehen, um Sperma zu befördern. Diesbezüglich wird ein rohrförmiger Körper gezeigt, der eine Samenkammer und eine Expansionskammer umfasst. Zur Trennung von Sperma- und Expansionskammer ist ein Stößel verschiebbar ausgebildet. Weiterhin ist ein Behälter in Verbindung mit der Expansionskammer stehend, um eine Flüssigkeitsmenge zu liefern. Darüber hinaus ist eine verschließbare Öffnung gezeigt, um Flüssigkeiten sowohl in dem Behälter als auch in die Expansionskammer einzubringen und zu entnehmen.

Ein tassenförmiges Spermienspeichergefäß, das am Eingang des Gebärmutterhalses anordenbar ist, zeigt die US 2013/0012766 A1.

Bei den geschilderten Dokumenten des Standes der Technik sind die dort gezeigten Inseminationsadapter so ausgebildet, dass für eine zielführende Füllung erhebliche Volumen von Ejakulat bzw. Sperma benötigt werden.

Bereits zum Füllen von Pumpreservoirs und Zuführungsschläuchen werden größere Mengen benötigt, die dem eigentlichen Zweck, nämlich dem Eindringen in die Gebärmutter und Eileiter nicht mehr zur Verfügung stehen.

Aus dem Vorgenannten ist es daher Aufgabe der Erfindung, eine weiterentwickelte Vorrichtung zur intracervicalen Insemination anzugeben, welche mit geringen Spermamengen einen hinreichenden Befruchtungserfolg liefert, welche einfach handhabbar ist, in der Herstellung sich als kostengünstig erweist und die auch bei unterschiedlichen Größen von Gebärmutterhälsen passgenau ist und einen sicheren Sitz schafft.

Die Lösung der Aufgabe der Erfindung erfolgt mit einer Vorrichtung zur intracervicalen Insemination gemäß der Merkmalskombination nach Anspruch 1, wobei die Unteransprüche mindestens zweckmäßige Ausgestaltungen und Weiterbildungen umfassen.

Es wird demnach von einer Vorrichtung zur intracervicalen Insemination ausgegangen, welche aus einer schalenförmigen, auf den Gebärmutterhals aufsetzbaren Kappe besteht. Die Kappe umfasst eine mit der Kappe verbundene, von dieser aufsteigenden Lanze zur Aufnahme und Führung von Sperma in den Gebärmutterhals.

Erfindungsgemäß besteht die Kappe aus einem elastischen Material, insbesondere einem elastischen Kunststoffmaterial.

Am zur Kappe weisenden Ende der Lanze ist in der Kappe eine integrale Ausbauchung zur Bildung eines integrierten Spermareservoirs vorgesehen.

Die Lanze umgreift mit ihrem kappenseitigen Ende den hierbei gebildeten Ausbauchungs-Reservoirraum unter Freilassung eines Spaltes.

Der Spalt ist in Längsrichtung bis zum kappenfernen Ende geführt und zum Austritt von Sperma ausgebildet, derart, dass Spermien unter Nutzung des vom Zervikalmukus ausgehenden kapillaren Sogs sicher und mit großer Wahrscheinlichkeit in den Gebärmutterhals diffundieren.

In einer bevorzugten Ausführungsform ist der Ausbauchungs-Reservoirraum exzentrisch versetzt bezogen auf den Bodenmittelpunkt einer halbkugelförmigen Kappe ausgeführt.

In Weiterbildung der Erfindung ist die Länge der Lanze größer oder gleich der Tiefe der jeweiligen Kappe.

Bei Ausbildung einer halbkugelförmigen Kappe ist diese bevorzugt schräg angeschnitten, wobei die durch den Schrägschnitt gebildete Ebene mit der Längsausdehnung der Lanze einen Winkel einschließt, welcher von 90° abweicht.

In einer Ausführungsform der Erfindung geht am rückseitigen Ende des Ausbauchungs-Reservoirraumes selbiger in einen Aufnahmefortsatz für ein chirurgisches Instrument, insbesondere eine Intubationszange über. Dieser Aufnahmefortsatz kann zum Beispiel als Nippel mit Griffvertiefung für das Instrument realisiert sein.

Die Kappe weist weiterhin an ihrer nicht mit dem Gebärmutterhals in Kontakt kommenden Außenseite einen Ansatz in Form z.B. einer Lasche auf, der dem Fixieren eines Fadens zum Entfernen der Vorrichtung dient.

In Weiterbildung der Erfindung weist die Lanze die Form eines langgestreckten spitzen Hohlkegelstumpfes auf, wobei der Spalt in Hohlkegellängsrichtung verläuft und einen Öffnungsschlitz eines durch den Hohlkegel realisierten Kanals bildet.

Der Fuß des Hohlkegelstumpfes schließt hierbei unmittelbar an das Ausbauchungsreservoir an.

In einer bevorzugten Ausgestaltung weist die Spitze des Hohlkegelstumpfes einen zum Schlitz hin gerichteten Schrägschnitt auf.

Im Hinblick auf den im Hohlkegelstumpf gebildeten Kanal nebst Schlitz und der Schrägschnitt-Ausbildung der Kappe ist der Fortsatz zur Aufnahme des Instrumentes so ausgebildet, dass ein gerichtetes Einführen der Vorrichtung im Sinne des Erzielens eines optimalen Behandlungserfolges gewährleistet wird.

Die Erfindung soll nachstehend anhand eines Ausführungsbeispieles sowie unter Zuhilfenahme von Figuren näher erläutert werden.

Hierbei zeigen:
- Fig. 1: eine perspektivische Darstellung der erfindungsgemäßen Vorrichtung in Vorderansicht;
- Fig. 2: eine Darstellung ebenfalls perspektivisch, jedoch schräg von oberhalb;
- Fig. 3: eine Darstellung ähnlich der Figuren 1 und 2, jedoch in schräger Seitenansicht sowie
- Fig. 4: eine Darstellung in Seitenansicht mit Fortsatz zum Ansetzen eines Instrumentes nebst Ansatz zum Fixieren eines Fadens zum Entfernen der Vorrichtung.

Die erfindungsgemäße Vorrichtung gemäß dem Ausführungsbeispiel und den Figuren dient der intracervicalen Insemination und besteht vom Grundsatz her aus einer schalenförmigen, auf den Gebärmutterhals aufsetzbaren Kappe 1.

Von der Kappe ausgehend erstreckt sich eine mit dieser verbundene, aufsteigende Lanze 2 zur Aufnahme und Führung von Sperma in den Gebärmutterhals (letzteres nicht gezeigt).

Die Kappe 1 besteht bevorzugt aus einem elastischen Kunststoffmaterial.

Am zur Kappe 1 weisenden Ende der Lanze ist in der Kappe 1 eine integrale Ausbauchung 3 zur Bildung eines Spermareservoirs vorgesehen.

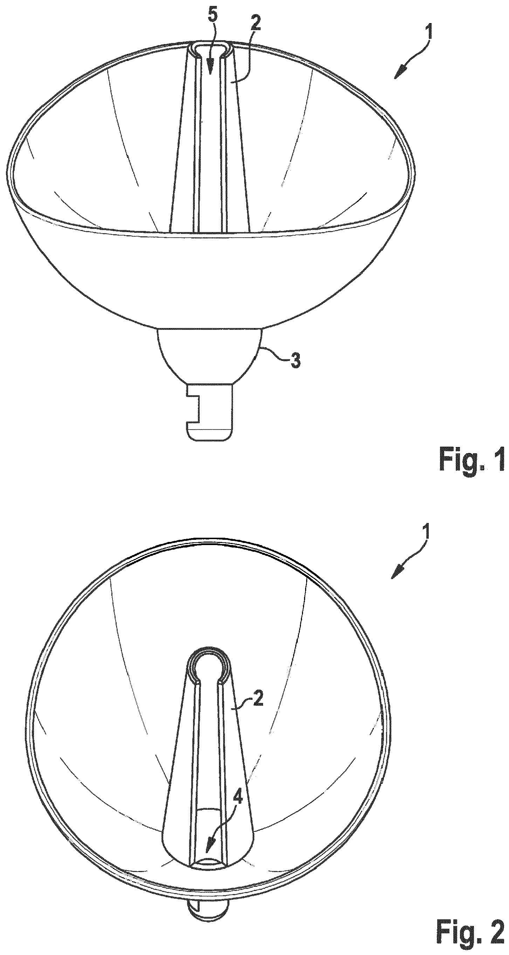

Das Spermareservoir 4 ist in der Figur 2 durch die Pfeildarstellung symbolisiert.

Die Lanze 2 umgreift mit ihrem kappenseitigen Ende den Ausbauchungs-Reservoirraum bzw. das Spermareservoir 4, was beispielsweise in der Figur 2 deutlich wird.

Darüber hinaus ist ein Spalt 5 gebildet, der in Längsrichtung der Lanze 2 verläuft. Der Spalt 5 dient der Führung und dem Austritt von Sperma derart, dass Spermien unter Nutzung des vom Zervikalmukus ausgehenden kapillaren Soges in den Gebärmutterhals bei entsprechend aufgesetzter Vorrichtung diffundieren können.

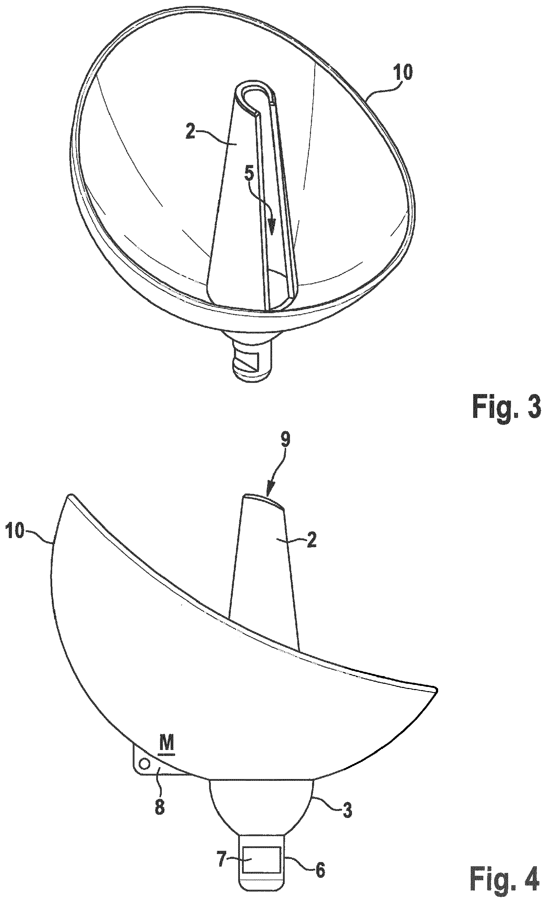

Die Figuren 3 und 4 zeigen eine bevorzugte Ausführungsform der Erfindung, wobei der Ausbauchungs-Reservoirraum 3 exzentrisch versetzt bezogen auf den Bodenmittelpunkt M einer halbkugelförmigen Kappe 10 ausgebildet ist.

Hier ist auch ersichtlich, dass die Länge der Lanze 2 größer oder gleich der Tiefe der Kappe 10 gewählt ist.

Die halbkugelförmig ausgebildete Kappe 10 ist quasi schräg angeschnitten, wobei die durch den Schrägschnitt gebildete Ebene mit der Längsausdehnung der Lanze 2 einen Winkel einschließt, welcher von 90° abweicht, wie in der Figur 4 dargestellt.

Am rückseitigen Ende des Ausbauchungs-Reservoirraumes 3 geht selbiger in einen Aufnahmefortsatz 6 für ein Instrument über.

Hierbei kann es sich um einen Aufnahmefortsatz 6 mit Grifffläche 7 für eine Intubationszange handeln.

Die Kappe 10 weist an ihrer nicht mit dem Gebärmutterhals in Kontakt kommende Außenseite einen Ansatz 8 zum Fixieren eines Fadens auf. Der Faden dient dem Entfernen der Vorrichtung, beispielsweise nach einer Zeit von zehn Stunden. Das Entfernen kann mit Hilfe des Fadens von der Patientin selbst vorgenommen werden, ohne dass ärztliche Hilfe notwendig ist.

Insbesondere aus den Darstellungen nach Figur 2 und 3, aber auch im Hinblick auf die Seitenansicht nach Figur 4 ist erkennbar, dass die Lanze 2 die Form eines langgestreckten, spitzen Hohlkegelstumpfes besitzt.

Der Spalt 5 verläuft in Kegellängsrichtung und sichert die Bildung eines Öffnungsschlitzes eines durch den Hohlkegel realisierten Kanals.

Der Fuß des Hohlkegelstumpfes grenzt unmittelbar an die Öffnungsseite des Ausbauchungsreservoirs 3 an (siehe Figur 3).

Die Spitze des Hohlkegelstumpfes weist einen zum Schlitz 5 hin gerichteten Schrägschnitt 9 auf.

Der Vorteil der erfindungsgemäßen Vorrichtung besteht unter anderem darin, dass die in den Gebärmutterhals hineinreichende Lanze 2 auf der Schlitzseite geöffnet ist. Hierdurch können Spermien leicht in den Zervikalmukus diffundieren. Dieser Diffusionsprozess wird unterstützt durch den kapillaren Sog, der vom Zervikalmukus ausgeht.

Die Schrägform der bevorzugt elastischen Halbschale sichert eine exakte Positionierung auch bei unterschiedlichen Größen von Gebärmutterhälsen im Vergleich zu starren oder halbstarren Vorrichtungen.

Die erfindungsgemäße Vorrichtung zur Insemination ist in verschiedenen Ausführungsformen herstellbar. Diesbezüglich wird die Dicke der Lanze, respektive der Umfang des Konus, je nach Größe des Zervikalkanals der zu behandelnden Person angepasst, entweder im Durchmesser reduziert oder kräftiger ausgeführt. Insofern wird den jeweiligen anatomischen Gegebenheiten Rechnung getragen.

Die erfindungsgemäße Vorrichtung kann ergänzt werden durch ein Instrument zur Selbstinsemination, zum Beispiel bei lesbischen Paaren mit Kinderwunsch.

Das Instrument zur Selbstinsemination kann im Bereich des Aufnahmefortsatzes 6 mit Hilfe der dortigen Ausnehmung 7 angesetzt werden, so dass die Handhabung beim Einsetzen erleichtert ist.

## Patentansprüche

1. Vorrichtung zur intracervicalen Insemination, bestehend aus einer schalenförmigen, auf den Gebärmutterhals aufsetzbaren Kappe (1) sowie mit einer mit der Kappe (1) verbundenen, von dieser aufsteigenden Lanze (2) zur Aufnahme und Führung von Sperma in den Gebärmutterhals,
**dadurch gekennzeichnet, dass**
die Kappe (1) aus einem elastischen Kunststoffmaterial besteht und am zur Kappe (1) weisenden Ende der Lanze (2) in der Kappe eine integrale Ausbauchung (3) zur Bildung eines Spermareservoirs (4) vorgesehen ist, wobei die Lanze (2) mit ihrem kappenseitigen Ende den Ausbauchungs-Reservoirraum (4) unter Freilassung eines Spaltes (5) umgreift und der Spalt in Längsrichtung bis zum kappenfernen Ende zur Führung und zum Austritt von Sperma ausgebildet ist derart, dass Spermien unter Nutzung des vom Zervikalmukus ausgehenden kapillaren Sogs in den Gebärmutterhals diffundieren.

2. Vorrichtung nach Anspruch 1,
**dadurch gekennzeichnet, dass**
der Ausbauchungs-Reservoirraum (3) exzentrisch versetzt bezogen auf den Bodenmittelpunkt (M) einer halbkugelförmigen Kappe (10) ausgebildet ist.

3. Vorrichtung nach Anspruch 1 oder 2,
**dadurch gekennzeichnet, dass**
die Länge der Lanze (2) größer oder gleich der Kappentiefe gewählt ist.

4. Vorrichtung nach Anspruch 2 oder 3,
**dadurch gekennzeichnet, dass**
die halbkugelförmig ausgebildete Kappe (1) schräg angeschnitten ist, wobei die durch den Schrägschnitt gebildete Ebene mit der Längsausdehnung der Lanze (2) einen Winkel einschließt, welcher von 90° abweicht.

5. Vorrichtung nach einem der vorangegangenen Ansprüche,
**dadurch gekennzeichnet, dass**
am rückseitigen Ende des Ausbauchungs-Reservoirraumes selbiger in einen Aufnahmefortsatz (6) für ein Instrument, insbesondere eine Intubationszange übergeht.

6. Vorrichtung nach einem der vorangegangenen Ansprüche,
**dadurch gekennzeichnet, dass**
die Kappe (1) an ihrer nicht mit dem Gebärmutterhals in Kontakt kommenden Außenseite einen Ansatz (8) zum Fixieren eines Fadens zum Entfernen der Vorrichtung besitzt.

7. Vorrichtung nach einem der vorangegangenen Ansprüche,
**dadurch gekennzeichnet, dass**
die Lanze (2) die Form eines langgestreckten, spitzen Hohlkegelstumpfes aufweist, wobei der Spalt (5) in Kegellängsrichtung verläuft und einen Öffnungsschlitz eines durch den Hohlkegel realisierten Kanals bildet.

8. Vorrichtung nach Anspruch,
**dadurch gekennzeichnet, dass**
der Fuß des Hohlkegelstumpfes unmittelbar an das Ausbauchungsreservoir bzw. den Ausbauchungs-Reservoirraum angrenzt.

9. Vorrichtung nach Anspruch 7 oder 8,
**dadurch gekennzeichnet, dass**
die Spitze des Hohlkegelstumpfes einen zum Schlitz (5) gerichteten Schrägschnitt (9) aufweist.
